# EUROPÄISCHE PATENTSCHRIFT

(11) **EP 2 106 333 B1**
(45) Veröffentlichungstag und Bekanntmachung des Hinweises auf die Patenterteilung: **02.01.2019**
(21) Anmeldenummer: 07818832.3
(22) Anmeldetag: 09.10.2007
(51) Int. Cl.: B29C 45/14, A61M 5/31, B29C 45/26, B29L 31/00

(54) **SPRITZENKÖRPER**
SYRINGE BODY
CORPS DE SERINGUE

(30) Priorität: 09.10.2006 DE 102006047670
(43) Veröffentlichungstag der Anmeldung: 07.10.2009
(73) Patentinhaber: G.B. BOUCHERIE, N.V., 8870 Izegem (BE)
(72) Erfinder: BOUCHERIE, Bart, Gerard, 8870 Izegem (BE)
(74) Vertreter: Prinz & Partner mbB
(86) Internationale Anmeldenummer: PCT/EP2007/008759
(87) Internationale Veröffentlichungsnummer: WO 2008/043516

(56) Entgegenhaltungen:
- EP-A- 0 382 126
- EP-A- 1 190 727
- US-A- 6 120 481

## Beschreibung

Die Erfindung betrifft ein Verfahren zur Herstellung eines hohlzylinderförmigen Injektionsspritzenkörpers durch Spritzgießen sowie einen durch dieses Verfahren hergestellten Injektionsspritzenkörper. Der Spritzenkörper ist mit einer in Längsrichtung angebrachten Skala versehen. Die Erfindung betrifft ferner eine Spritzgießform zur Herstellung eines Injektionsspritzenkörpers.

Injektionsspritzen bestehen aus einem Spritzenkörper und einem darin beweglichen Kolben. Insbesondere bei den sogenannten Einwegspritzen besteht der Spritzenkörper aus einem Kunststoff und wird kostengünstig durch Spritzgießen als Massenartikel hergestellt. Zum Dosieren des zu injizierenden Medikaments muß der Injektionsspritzenkörper mit einer in Längsrichtung angebrachten Skala versehen sein.

Die Meßskala kann durch Bedrucken aufgebracht werden. Die Meßskala kann alternativ von außen auf den Spritzenkörper aufgeklebt werden. In beiden Fällen ist ein Arbeitsschritt im Anschluß an den Spritzgießprozeß erforderlich.

Aus der US 6,120,481 A ist ein Spritzenkörper bekannt, der eine mit einer Skala bedruckten Folie aufweist. Die Folie ist im Spritzenkörper eingebettet.

Da Injektionsspritzen Massenartikel sind, besteht ein Bedarf an einem Verfahren, mit dem Injektionsspritzenkörper ohne Aufwand mit einer Skala versehen werden können.

Die Erfindung stellt ein Verfahren mit den Merkmalen des Anspruchs 1 bereit, bei dem eine Skala zunächst auf einer dünnen Trägerfolie aufgebracht wird und die Trägerfolie mit der Skala vor dem Spritzen in die Spritzgießform eingelegt wird wobei die Trägerfolie von der Kunststoffmasse an die Innenwand der Spritzgießform angedrückt wird. Das Bedrucken von Folie ist eine gut beherrschte Technik, mit der schnell eine dauerhafte Bedruckung erreicht wird. Das Einlegen der Trägerfolie in die Gießform kann leicht in den Gußprozeß integriert werden und erfolgt beispielsweise unmittelbar nachdem der jeweils zuvor gefertigte Spritzenkörper entformt wurde.

Vorzugsweise ist in die formgebende Fläche der Spritzgießform eine Ausrichtstruktur eingearbeitet, mit deren Hilfe die Trägerfolie präzise positioniert wird. Die Ausrichtstruktur ist dabei in einer bevorzugten Ausführungsform eine flache Ausnehmung, deren Form der Außenkontur der Trägerfolie entspricht, vorzugsweise ist die Ausnehmung einen Bruchteil der Dicke der Trägerfolie tief. Die eingelegte Trägerfolie ragt also beim Spritzvorgang in die Kunststoffmasse hinein und wird so am fertigen Injektionsspritzenkörper sicher gehalten.

Alternativ kann in die formgebende Fläche der Spritzgießform wenigstens ein Anschlag eingearbeitet sein, an dem die Trägerfolie ausgerichtet wird. Dieser Anschlag ist vorzugsweise eine Stufe, so daß die Trägerfolie bis zur Anlage an dieser Stufe geschoben werden kann.

Bei einer vorteilhaften Ausführungsform wird die Trägerfolie vor dem Einlegen in die Spritzgießform entsprechend deren Innenradius vorgeformt.

Beim Spritzgießen wird der Kunststoff in flüssiger Form in die Spritzgießform gespritzt. Der Strom des flüssigen Kunststoffs kann die eingelegte Trägerfolie gegen ein Verschieben während des Einspritzvorgangs sichern, wenn der Einspritzort in Bezug auf die Trägerfolie geschickt gewählt wird. Vorzugsweise ist die Trägerfolie mit einer Öffnung ausgebildet, und die Öffnung ist mit dem Anspritzkanal fluchtend angeordnet, so daß beim Spritzen die eingespritzte Kunststoffmasse die Öffnung durchquert. Die eingespritzte Kunststoffmasse umströmt dann den Kern der Spritzgießform zuerst in einer Richtung weg von der Trägerfolie und baut dann einen Druck auf, durch den die Trägerfolie an die Innenfläche der Form angepreßt und somit lagefixiert wird.

Weitere Vorteile des erfindungsgemäßen Verfahrens werden durch die nachstehende Beschreibung bevorzugter Ausführungsformen anhand der beigefügten Zeichnung deutlich. Hierbei zeigen:
- Figur 1 einen nach dem erfindungsgemäßen Verfahren hergestellten Injektionsspritzenkörper;
- Figur 2 eine dünne Trägerfolie mit aufgebrachter Skala;
- Figur 3 eine teilweisen Querschnitt durch eine Spritzgießform mit eingelegter Trägerfolie in einer Ausnehmung;
- Figur 3a eine Detailvergrößerung der Figur 3 zur Verdeutlichung der Ausnehmung
- Figur 4 eine teilweisen Querschnitt durch einen Injektionsspritzenkörper gemäß einer weiteren Ausführungsform mit Trägerfolie an einem Anschlag;
- Figur 4a eine Detailvergrößerung der Figur 4 zur Verdeutlichung einer Stufe;
- Figur 5 eine schematische Darstellung der Positionierung der Einspritzdüse im Verhältnis zum fertigen Injektionsspritzenkörper.

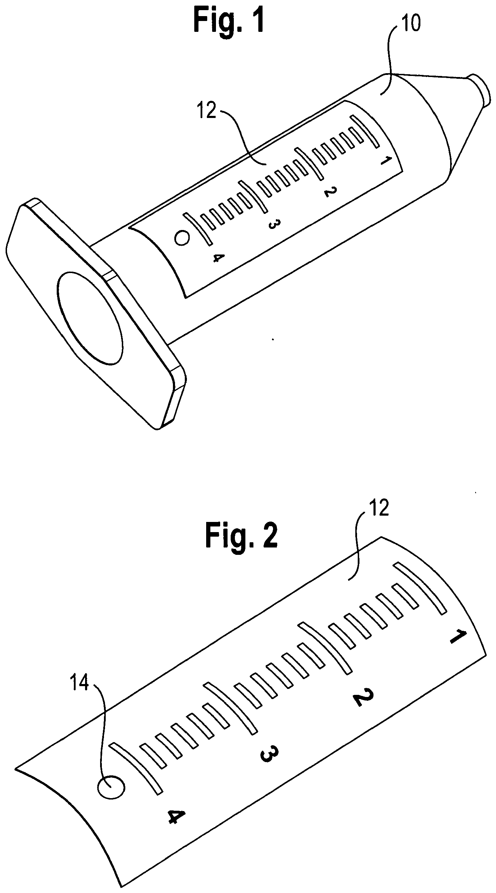

Figur 1 zeigt einen Injektionsspritzenkörper 10 mit einer Trägerfolie 12, auf der eine Skala angebracht ist. Der Injektionsspritzenkörper 10 ist hohlzylinderförmig und weist an einer Seite eine konische Verjüngung auf, an der eine Injektionsnadel angebracht wird. An seinem anderen Ende ist ein Flansch zu erkennen, der die spätere Handhabung der Spritze erleichtert. Der Injektionsspritzenkörper kann an seinen Enden auch andere Formen annehmen und beispielsweise auch keinen Flansch aufweisen, entscheidend für die Erfindung sind nur die Hohlzylinderform und die aufgebrachte Skala. Die Breite der Trägerfolie 12 beträgt etwa ein Drittel bis ein Viertel des Umfangs des Spritzenkörpers. Die Länge des Injektionsspritzenkörpers beträgt etwa zwischen 5 und 15 cm, sein Durchmesser liegt entsprechend etwa zwischen 1 und 3 cm.

Figur 2 zeigt die Trägerfolie 12, auf der bereits die Skala beispielsweise durch Siebdruck aufgebracht ist. An einem Ende, unter der Maßzahl 4, ist eine Öffnung 14 zu erkennen. Auf die Bedeutung der Öffnung 14 wird weiter unten noch eingegangen. Die dargestellte Trägerfolie 12 ist bereits vorgeformt, so daß sie sich besser an die Innenwand einer Spritzgießform anschmiegt.

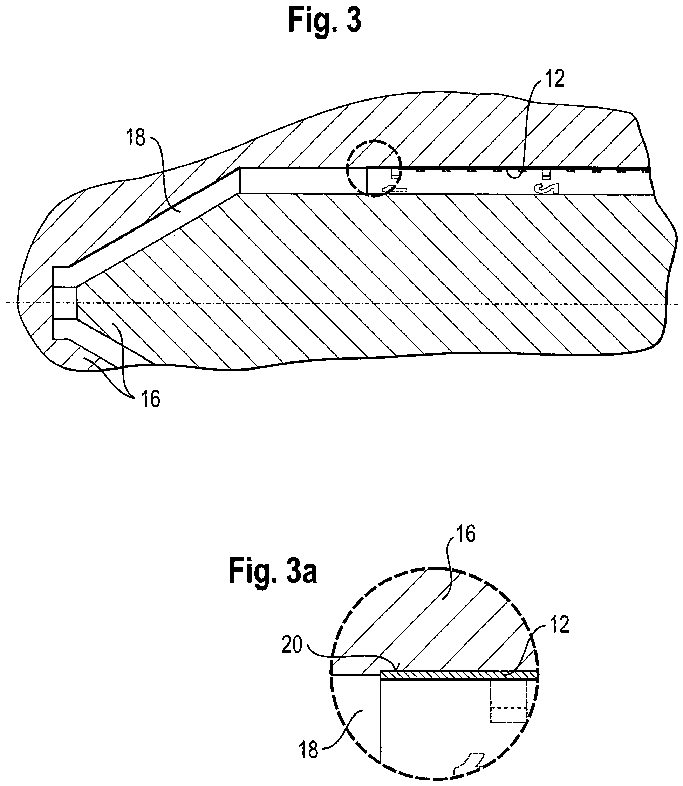

Figur 3 zeigt ausschnittsweise einen Querschnitt durch eine Spritzgießform 16. Ein Hohlraum 18 hat die Form des herzustellenden Injektionsspritzenkörpers 10. Es ist die bereits eingelegte Trägerfolie 12 zu erkennen. In der Figur 3a ist eine Detailvergrößerung aus Figur 3 zu sehen. Im oberen Teil ist der Schnitt durch die Spritzgießform 16 dargestellt. Die Trägerfolie 12, die ebenfalls angeschnitten ist, liegt in einer Ausnehmung 20. Wie in der Detailvergrößerung zu erkennen, ragt sie in den Hohlraum 18 hinein, da die Tiefe der Ausnehmung nur einem Bruchteil der Dicke der Trägerfolie 12 entspricht.

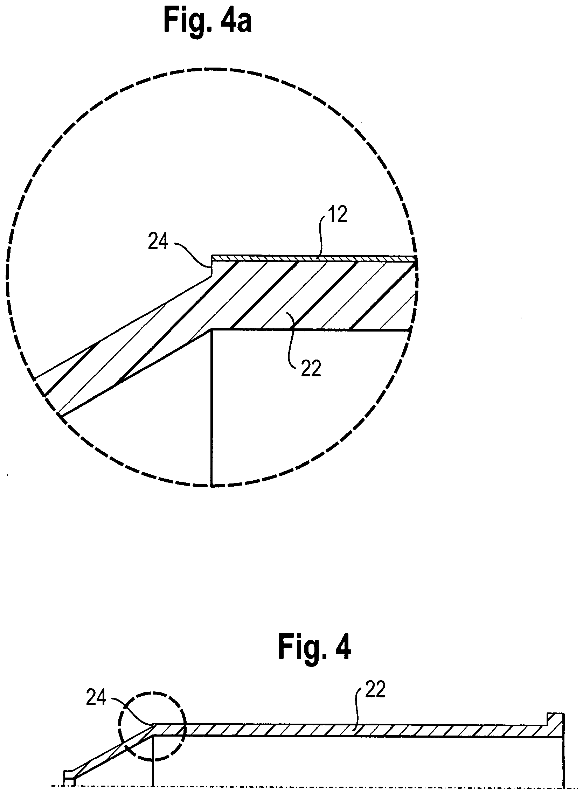

Figur 4 zeigt einen Schnitt durch einen Injektionsspritzenkörper 22 einer anderen Ausführungsform. Erst in der Detailvergrößerung in Figur 4a ist die aufgebrachte Trägerfolie 12 zu erkennen. An der Stelle, an der der hohlzylinderförmige Teil des Injektionsspritzenkörpers 22 in eine konische Form übergeht, liegt eine Stufe 24. Ein Rand der Trägerfolie 12 fluchtet mit der Stufe 24. Die entsprechende inverse Stufe der Spritzgießform, mit der Injektionsspritzenkörper 22 hergestellt wird, dient als Anschlag für die Trägerfolie 12 beim Einlegen derselben in die Form.

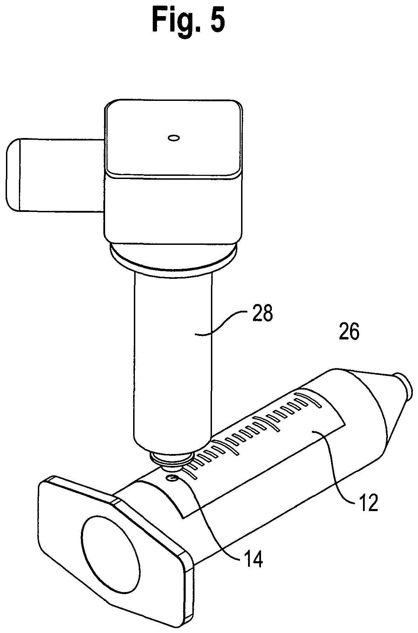

Neben der Ausnehmung 20 bzw. der Stufe 24 hilft die Wahl des Einspritzpunktes, ein Wegspülen oder Verrutschen der Trägerfolie 12, die nicht die ganze formgebende Fläche der Spritzgießform bedeckt, zu vermeiden. In Figur 5 ist der bevorzugte Einspritzpunkt symbolisch dargestellt. Über einem Injektionsspritzenkörper 26, der dem Injektionsspritzenkörper 10 oder dem Injektionsspritzenkörper 22 entsprechen kann, ist eine Spritzdüse 28 schematisch dargestellt. Für eine bessere Übersichtlichkeit ist die Spritzgießform nicht dargestellt, in der der Injektionsspritzenkörper 26 spritzgegossen wird. Wie bereits in der Beschreibung zu Figur 2 erwähnt, weist die Trägerfolie 12 eine Öffnung 14 auf, die Öffnung 14 ist mit dem Anspritzkanal der Spritzdüse 28 fluchtend angeordnet, so daß beim Spritzen die eingespritzte Kunststoffmasse die Öffnung 14 durchquert. Die Öffnung 14 liegt in der Nähe des dem Flansch zugewandten Randes der Trägerfolie 12 und im wesentlichen mittig zu ihren Längsrändern. Es bilden sich damit beim Spritzgießen in der Hohlform Strömungsverhältnisse aus, mit denen die Trägerfolie 12 nicht weggespült sondern im Gegenteil noch an die Innenwand der Spritzgießform angedrückt wird. Die unter Druck eingespritzte Kunststoffmasse umströmt zunächst den innenliegenden Kern und füllt als erstes den der Einspritzöffnung 14 gegenüber und auf der anderen Seite des Kerns liegenden Hohlraum, um dann von beiden Seiten des Kerns aus den Hohlraum in Richtung der Trägerfolie 12 auszufüllen. Der sich dabei aufbauende Druck preßt die Trägerfolie 12 an die Innenfläche der Spritzgießform.

## Patentansprüche

1. Verfahren zur Herstellung eines hohlzylinderförmigen Injektionsspritzenkörpers (10, 22, 26), der mit einer in Längsrichtung angebrachten Skala versehen ist, durch Spritzgießen, wobei die Skala auf einer dünnen Trägerfolie (12) aufgebracht wird und die Trägerfolie (12) mit der Skala vor dem Spritzen in die Spritzgießform (16) eingelegt wird, **dadurch gekennzeichnet, dass** die Trägerfolie (12) von der Kunststoffmasse an die Innenwand der Spritzgießform (16) angedrückt wird.

2. Verfahren nach Anspruch 1, **dadurch gekennzeichnet, daß** die Trägerfolie (12) in der Spritzgießform durch in deren formgebende Fläche eingearbeitete Ausrichtstrukturen präzise positioniert wird.

3. Verfahren nach Anspruch 2, **dadurch gekennzeichnet, daß** in die formgebende Fläche eine flache Ausnehmung (20) eingearbeitet ist, deren Form der Außenkontur der Trägerfolie (12) entspricht, und die Trägerfolie in die Ausnehmung eingelegt wird.

4. Verfahren nach Anspruch 3, **dadurch gekennzeichnet, daß** die Tiefe der Ausnehmung (20) einem Bruchteil der Dicke der Trägerfolie (12) entspricht.

5. Verfahren nach Anspruch 2, **dadurch gekennzeichnet, daß** in die formgebende Fläche wenigstens ein Anschlag (24) eingearbeitet und die Trägerfolie (12) an diesem Anschlag ausgerichtet wird.

6. Verfahren nach Anspruch 5, **dadurch gekennzeichnet, daß** der Anschlag als Stufe (24) ausgebildet wird.

7. Verfahren nach einem der vorstehenden Ansprüche, **dadurch gekennzeichnet, daß** die Trägerfolie (12) mit einer Öffnung (14) ausgebildet und die Öffnung (14) mit dem Anspritzkanal fluchtend angeordnet wird, so daß beim Spritzen die eingespritzte Kunststoffmasse die Öffnung (14) durchquert.

8. Verfahren nach einem der vorstehenden Ansprüche, **dadurch gekennzeichnet, daß** die Trägerfolie (12) vor dem Einlegen in die Spritzgießform vorgeformt wird.

9. Injektionsspritzenkörper, hergestellt durch das Verfahren nach einem der Ansprüche 1 bis 8.

10. Spritzgießform zur Herstellung eines Injektionsspritzenkörpers (10, 22, 26) mit einer formgebenden Fläche, **dadurch gekennzeichnet, daß** in die formgebende Fläche eine flache Ausnehmung (20) eingearbeitet ist, daß eine Einspritzöffnung in der flachen Ausnehmung liegt und daß die Form der Außenkontur der Ausnehmung einer Trägerfolie (12) entspricht, die in die flache Ausnehmung eingelegt wird.

## Claims

1. A method of producing, by injection molding, a hollow cylindrical syringe body (10, 22, 26) which is provided with a scale fitted in the longitudinal direction,
the scale being applied to a thin carrier film (12), and the carrier film (12) with the scale being inserted into the injection mold (16) prior to the injection molding process, **characterized in that**
the carrier film (12) is pressed against the inner wall of the injection mold (16) by the plastic composition.

2. The method according to claim 1, **characterized in that** the carrier film (12) is accurately positioned in the injection mold by alignment structures incorporated in the shaping surface of the injection mold.

3. The method according to claim 2, **characterized in that** the shaping surface has a flat recess (20) incorporated therein having a shape that corresponds to the outer contour of the carrier film (12), and the carrier film is inserted into the recess.

4. The method according to claim 3, **characterized in that** the depth of the recess (20) corresponds to a fraction of the thickness of the carrier film (12).

5. The method according to claim 2, **characterized in that** the shaping surface has at least one stop (24) incorporated therein and the carrier film (12) is aligned by means of this stop.

6. The method according to claim 5, **characterized in that** the stop is made in the form of a step (24).

7. The method according to any of the preceding claims, **characterized in that** the carrier film (12) is made to have an opening (14) and the opening (14) is arranged in alignment with the runner, so that in the injection molding process the plastic composition that is injected traverses the opening (14).

8. The method according to any of the preceding claims, **characterized in that** the carrier film (12) is preformed before being inserted into the injection mold.

9. A syringe body, produced by means of the method according to any of claims 1 to 8.

10. An injection mold for producing a syringe body (10, 22, 26) having a shaping surface, **characterized in that** the shaping surface has a flat recess (20) incorporated therein, that an injection opening is disposed in the flat recess, and that the shape of the outer contour of the recess corresponds to a carrier film (12) which is inserted into the flat recess.

## Revendications

1. Procédé de fabrication d'un corps de seringue à injection (10, 22, 26) en forme de cylindre creux pourvu d'une échelle graduée agencée dans le sens longitudinal par moulage par injection,
l'échelle graduée étant appliquée sur une feuille support (12) mince, et la feuille support (12) présentant l'échelle graduée étant insérée dans le moule d'injection (16) avant l'injection, **caractérisé en ce que**
la feuille support (12) est pressée contre la paroi intérieure du moule d'injection (16) par la masse de matière plastique.

2. Procédé selon la revendication 1, **caractérisé en ce que** la feuille support (12) est positionnée de manière précise dans le moule d'injection au moyen de structures d'alignement incorporées dans la face de façonnage de celui-ci.

3. Procédé selon la revendication 2, **caractérisé en ce qu'**un évidement plat (20) dont la forme correspond au contour extérieur de la feuille support (12) est incorporé dans la face de façonnage, et **en ce que** la feuille support est insérée dans l'évidement.

4. Procédé selon la revendication 3, **caractérisé en ce que** la profondeur de l'évidement (20) correspond à une fraction de l'épaisseur de la feuille support (12).

5. Procédé selon la revendication 2, **caractérisé en ce qu'**au moins une butée (24) est incorporée dans la face de façonnage et **en ce que** la feuille support (12) est alignée sur cette butée.

6. Procédé selon la revendication 5, **caractérisé en ce que** la butée est réalisée sous forme de gradin (24).

7. Procédé selon l'une des revendications précédentes, **caractérisé en ce que** la feuille support (12) est réalisée avec un orifice (14) et **en ce que** l'orifice (14) est agencé en alignement sur le canal de projection de sorte la masse de matière plastique injectée traverse l'orifice (14) lors de l'injection.

8. Procédé selon l'une des revendications précédentes, **caractérisé en ce que** la feuille support (12) est préformée avant l'insertion dans le moule d'injection.

9. Corps de seringue à injection fabriqué au moyen du procédé selon l'une des revendications 1 à 8.

10. Moule d'injection pour la fabrication d'un corps de seringue à injection (10, 22, 26), présentant une face de façonnage, **caractérisé en ce qu'**un évidement plat (20) est incorporé dans la face de façonnage, **en ce qu'**un orifice d'injection est ménagé dans l'évidement plat, et **en ce que** la forme du contour extérieur de l'évidement correspond à une feuille support (12) qui est insérée dans l'évidement plat.
